# EUROPEAN PATENT SPECIFICATION

(11) **EP 2 629 632 B1**
(45) Date of publication and mention of the grant of the patent: **14.03.2018**
(21) Application number: 11773458.2
(22) Date of filing: 21.10.2011
(51) Int. Cl.: A23L 19/00, A23L 33/175, A61P 9/00, A61P 13/10, A61K 31/198

(54) **FORMULATION COMPRISING ARGININE, USE AND PREPARATION THEREOF**
FORMULIERUNG MIT ARGININ, IHRE VERWENDUNG UND HERSTELLUNG
FORMULATION COMPRENANT DE L'ARGININE, SON UTILISATION ET SA PRÉPARATION

(30) Priority: 22.10.2010 US 405714 P
(43) Date of publication of application: 28.08.2013
(73) Proprietor: Piatti, Piermarco, 20824 Lazzate (IT); Monti, Lucilla, 20824 Lazzate (IT)
(72) Inventor: BOSI, Emanuele, I-20132 Milano (IT); MONTI, Lucilla Domenica, I-20132 Milano (IT); PIATTI, Piermarco, I-20132 Milano (IT); SETOLA, Emanuela, I-20132 Milano (IT); CASIRAGHI, Maria Cristina, I-20133 Milano (IT); PAGANI, Maria Ambrogina, I-20133 Milano (IT); QUAGLIA, Lucio, Padova (IT)
(74) Representative: Capasso, Olga
(86) International application number: PCT/EP2011/068471
(87) International publication number: WO 2012/052555

(56) References cited:
- EP-A1- 0 710 448
- WO-A1-99/59433
- US-A1- 2007 014 914

## Description

### Field of the invention

The invention relates to the formulation and to the technology of production of an edible product (*e.g*. biscuit, bar) containing at least 10 % (wet weight) of L-arginine.

### Background to the invention

Insulin resistance, endothelial dysfunction, and inflammation are important cardiovascular risk factors in coronary artery disease patients and L-arginine seems to have antiinflammatory and metabolic advantages in these patients (8). It was also showed that L-arginine is useful in atherosclerosis prevention in humans affected by coronary artery disease. Chronic L-arginine oral supplementation has been proven to have beneficial effects over endothelial function both in healthy individuals and in type 2 diabetic patients especially when it is associated with physical exercise (9). In addition, it was demonstrated that chronic L-arginine therapy added to a physical exercise and diet program could also improve glucose metabolism and insulin sensitivity in a population of obese type 2 diabetic patients and in patients with the Metabolic Syndrome. Furthermore, it improved endothelial function, oxidative status, and adipokine release (19). Long-term oral L-arginine treatment resulted in an additive effect compared with a diet and exercise training program alone on glucose metabolism and insulin sensitivity (18).

The use of L-arginine as food supplement to a normal diet, in relatively large doses, has been proved to have a salutary effect on cardiovascular diseases as extensively described in literature, both in animal studies and in humans (13,15). L-arginine was found to be bioavailable and effective in the prevention of impairment of glucose metabolism and endothelial dysfunction, improving blood flow.

The international patent application WO1999/059433 discloses the formulation of an healthy bar to provide an organoleptically acceptable preparation (12-14). The document reports health bars having at least 2 weight % of at least one of amino acids L-arginine and L-lysine in combination with from about 20 to 50 weight % of protein, 20 to 50 weight % of fruit as paste and solids, from 10 to 35 weight % of carbohydrates and 0 to 5 weight % of dietary fiber to form an organoleptically acceptable food supplement.

In WO1999/059433 (12), the bars are made by preparing a syrup, at an elevated temperature, adding a fruit paste and cooling, followed by the addition of minor ingredients, then the amino acids and a portion of the protein are added and mixed, finally the remaining ingredients are added and a bar is formed. Further, commercially available products (Heartbar®, Cooke Pharma, Belmont, CA, USA) were reported as inducing negative effects on endothelial function and platelet aggregations (1).

EP 0 710 448 and US 2007/014914 disclose cereal based dietary compositions including fruits, nuts and popped rice. In particular, EP 0 710 448 suggests rather high concentrations of nuts, fruits and popped rice. US 2007/014914 suggests that a large variety of ingredients can be added to food bars rich in proteinaceous material.

Because of the benefit of L-arginine, there is the need for a formulation having a high content (at least 10%) of L-arginine completely bioavailable (> 99%), being also palatable, homogeneous, having low amounts of sugar and optionally suitable for insulin-resistant and/or carbohydrate-intolerant subjects and that does not induce side effects on endothelial or other functions.

The present invention provides such formulation which may be produced by a technological process involving sonication.

### Brief Description of the invention

In the present invention, a uniformly shaped food product comprising a high content in L-arginine and a low content in sugars and proteins was obtained. In particular, the food product is produced by means of sonication, a process conducted at low temperature range, which prevents the degradation of the amino acid by Maillard reactions or other heat dependent modifications.

Therefore, the formulation of the invention containing high content of L-Arginine, is characterized by:
- low amount of calories;
- low amount of sugars;
- no high temperature employed to bind all ingredients;
- low content of proteins;
- full homogeneity of a combination of nutrients in the product;
- pleasant taste, by the presence of for instance candies orange slices or un-sugared dried blueberries, raspberry, blackberry;

The preparation process of the present invention comprises that all the ingredients (for instance puffed rice and whole wheat flakes, candies orange slices and granulated hazelnut) are mixed with L-arginine. Then, a step of sonication at low temperature is carried out. The present process allows obtaining a uniformly shaped food product.

The food product may be in the form of a biscuit or a bar snack. In particular, it contains at least 1 g of L-arginine per unit (about 10 g).

It is therefore an object of the invention an edible formulation comprising the following ingredients (% on mix):
- 19 to 30 weight % of a dietary supplement comprising at least 50 % of L-arginine or a physiologically acceptable salt thereof,
- 20 to 35 weight % of cereal flakes,
- 14 to 25 weight % of puffed brown or white rice,
- 12 to 24 weight % of nuts,
- 9 to 18 weight% of orange rind or dried fruit such as cranberries, blueberries, raspberry, blackberry
- 2 to 10 weight % of water and/or fruit juice.

Preferably the formulation comprises the following ingredients (% on mix):
- 20 to 25 weight % of a dietary supplement comprising at least 50 % of L-arginine or a physiologically acceptable salt thereof,
- 24 to 28 weight % of cereal flakes,
- 16 to 20 weight % of puffed brown or white rice,
- 15 to 19 weight % of nuts,
- 11 to 15 weight% of orange rind or dried fruit such as cranberries, blueberries, raspberry, blackberry
- 4 to 8 weight % of water and/or fruit juice.

In a preferred embodiment the edible formulation is in the form of a food product.

Preferably, the food product is in the form of a biscuit or a bar snack.

In a still preferred embodiment the edible formulation comprises in % wet weight
- at least 10 ww % of L-arginine or a physiologically acceptable salt thereof,
- 5 to 10 ww % of proteins,
- 25 to 32 ww % of starch,
- 6 to 8 ww % of sugars,
- 9 to 12 ww % of total fat,
- 4 to 7 ww % of total dietary fibers.

Preferably the formulation comprises in % wet weight:
- 11 to 15 ww % of L-arginine or a physiologically acceptable salt thereof,
- 5 to 6 ww % of proteins,
- 29 to 31 ww % of starch,
- 6 to 7 ww % of sugars,
- 9.5 to 11.5 ww % of total fat,
- 4.9 to 6.7 ww % of total dietary fibers.

Still preferably the formulation comprises in % wet weight:
- 11.3 to 11.5 ww % of L-arginine or a physiologically acceptable salt thereof,
- 5.0 to 5.4 ww % of proteins,
- 29.0 to 30.8 ww % of starch,
- 6.6 to 6.9 ww % of sugars,
- 9.6 to 11.4 ww % of total fat,
- 4.9 to 6.7 ww % of total dietary fibers.

In a preferred embodiment the starch is from cereals. In a yet preferred embodiment the sugars comprise less than 2 % of fructose.

Preferably, the edible formulation of the invention as described above is for use as a dietary supplement.

Preferably, the edible formulation of the invention as described above is for use in the treatment and/or prevention of metabolic syndrome or as a co-adjuvant for the treatment and/or prevention of a metabolic syndrome or for use in the treatment and/or prevention of a pathology wherein the loss of weight and/or fat mass is desirable, wherein said metabolic syndrome includes patients with insulin resistance syndrome, hyperinsulinemia, a population at highly increased cardiovascular risk.

Still preferably, the edible formulation of the invention as described above is for use in the treatment and/or prevention of obesity, favoring body weight reduction with positive effects on fat mass within a hypocaloric diet.

In a preferred embodiment the obesity is associated with impaired glucose tolerance and metabolic syndrome.

Still preferably, the edible formulation of the invention as described above is for use in subjects with or at risk of cardiovascular disease, endothelial dysfunction, altered blood pressure, metabolic Syndrome (including patients with insulin resistance syndrome, hyperinsulinemia, a population at highly increased cardiovascular risk), high level of triglycerides, low level of HDL cholesterol, obesity, and impaired glucose tolerance or diabetes.

It is a further object of the invention a process for the preparation of the edible formulation as described above comprising:
(a) mixing all the ingredients;
(b) adding water and/or fruit juice;
(c) sonicating the mixture at a temperature between 25 - 40 °C;
(d) optionally, drying the sonicated mixture.

Preferably the sonication is carried out at 20 to 40 kHz. Still preferably the sonication is carried out for 20 - 2000 milliseconds.

Yet preferably the drying step is performed at a temperature less than 60 °C.

Cereal flakes may be provided as a mixture of corn, oat, whole wheat or other cereal flakes. The nuts may be hazelnut, almond, pine nut or other nuts.

In the present invention metabolic syndrome is a cluster of metabolic abnormalities which includes diabetes or impaired glucose tolerance, hypertension, dyslipidemia, obesity and increased risk of cardiovascular disease (7). It affects one in five people, and prevalence increases with age. Some studies estimate the prevalence in the USA to be up to 25% of the population (3).

Metabolic syndrome is also known as metabolic syndrome X, syndrome X, insulin resistance syndrome, Reaven's syndrome (named for Gerald Reaven), and CHAOS (in Australia, 20).

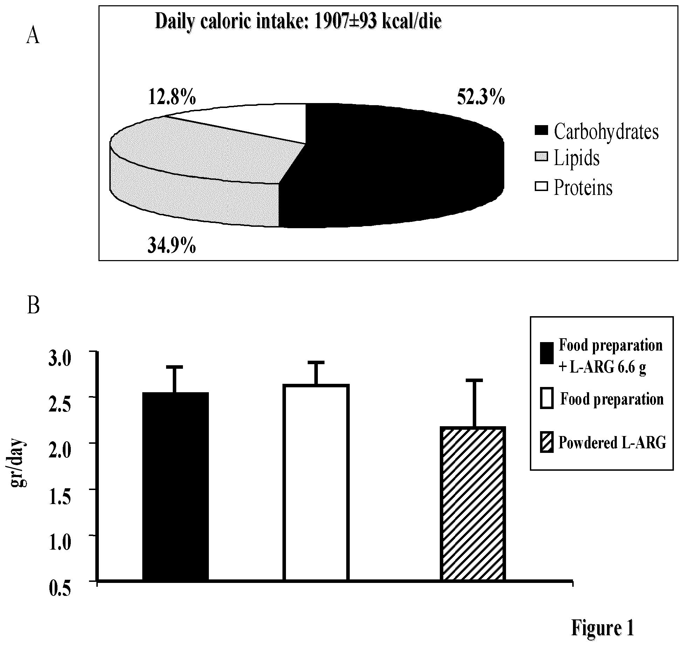

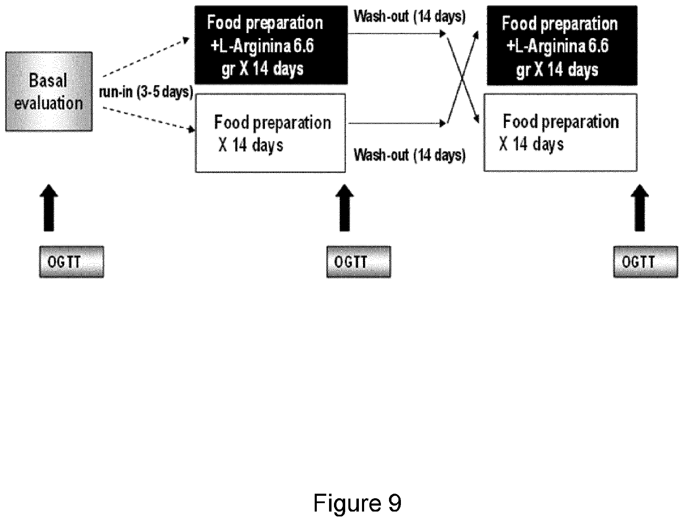

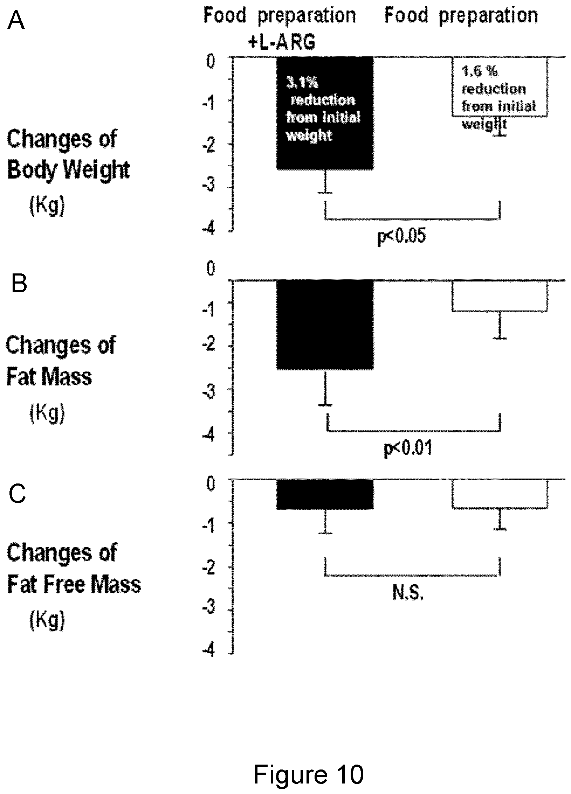

The invention will be now illustrated by means of non-limiting examples. Figures 1 to 8 refer to studies in healthy subjects while figures 9 to 23 show the beneficial effects of the invention in obese subjects with impaired glucose tolerance (IGT) and metabolic syndrome (MS).
**Figure 1****:** A) Mean daily caloric intake of the healthy subjects involved in the study, separated in carbohydrate, lipid and protein percentages, B) L-Arginine mean dietary intake in the healthy subjects involved in the study during the three days preceding tests represented in g/day. There is no difference between the groups receiving powdered L-Arginine, the food supplement of the invention containing L-Arginine or a food supplement without L-Arginine. Data are presented as Mean±SD.

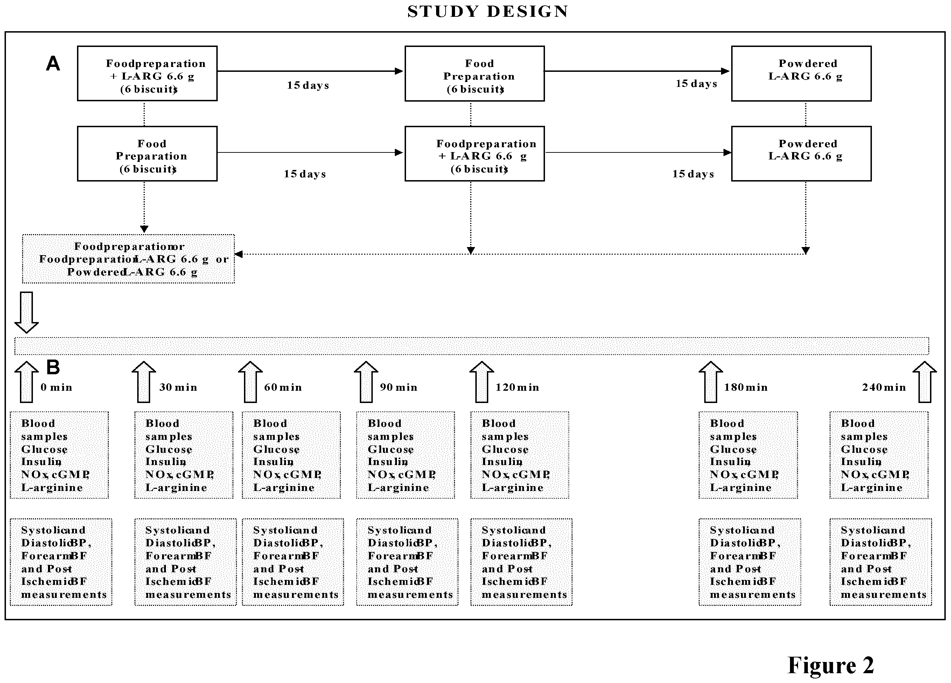

**Figure 2****:** Study design. **A.** Each subject performed three tests: food preparation (6 biscuits) with the addition of L-Arginine (6.6 g), the same food preparation (6 biscuits) prepared without the addition of L-Arginine or to 6.6 g of powdered L-Arginine. **B.** Time course of blood samples during each test and the parameters under evaluation are reported for each time. In particular, glucose, insulin, NOx and L-arginine levels were evaluated at 0, 30, 60, 90, 120, 180 and 240 minutes. c-GMP, forearm blood flow (BF) and post-ischemic BF were evaluated at 0, 60, 120 , 180 and 240 minutes. Systolic and diastolic blood pressure (BP) was evaluated at 0, 120, 180 and 240 minutes.

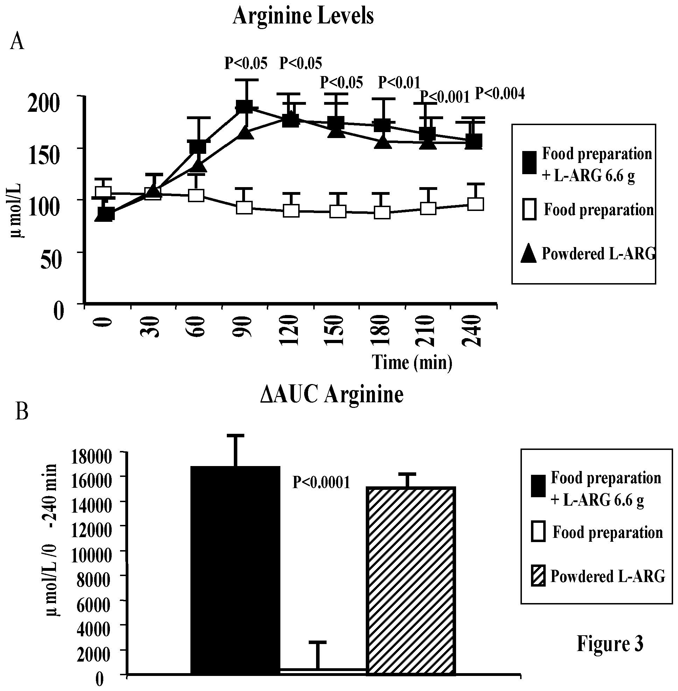

**Figure 3****:** A) Plasma L-Arginine levels in patients receiving the formulation of the invention (black squares), 6.6 g of powdered L-Arginine (triangles) or the biscuit not containing L-Arginine (white squres) and B) Incremental area under the curve for L-Arginine (ΔAUC L-Arginine), calculated using the trapezoidal rule, according to the different treatment groups. Data are presented as Mean±SD.

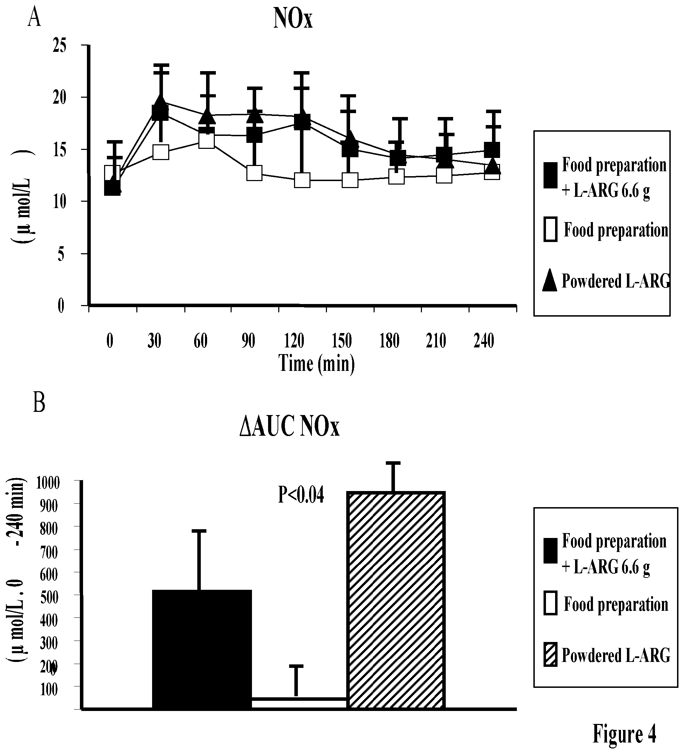

**Figure 4****:** A) Plasma NOx levels in subjects receiving the three different treatment conditions and B) Incremental area under the curve for NOx (ΔAUC NOx), calculated using the trapezoidal rule. Data are presented as Mean±SD.

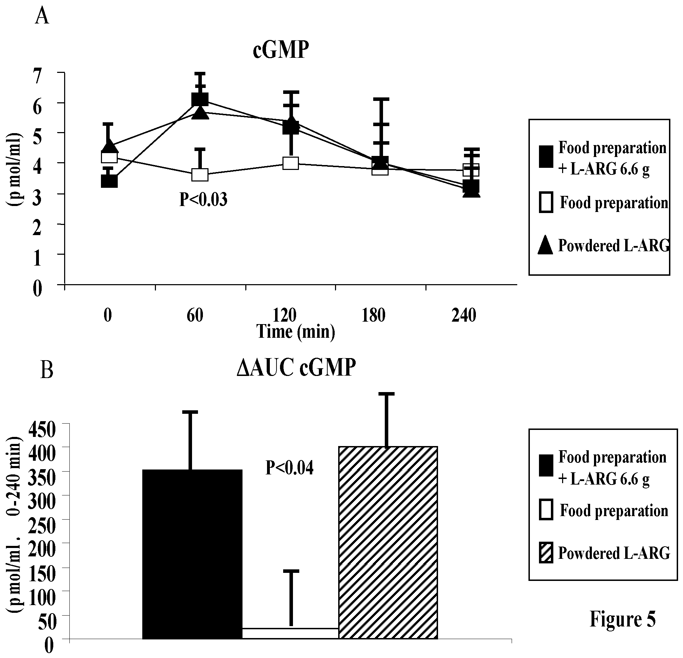

**Figure 5****:** A) Plasma cGMP levels in subjects receiving the three different treatment conditions and B) Incremental area under the curve for cGMP (ΔAUC cGMP), calculated using the trapezoidal rule. Data are presented as Mean±SD.

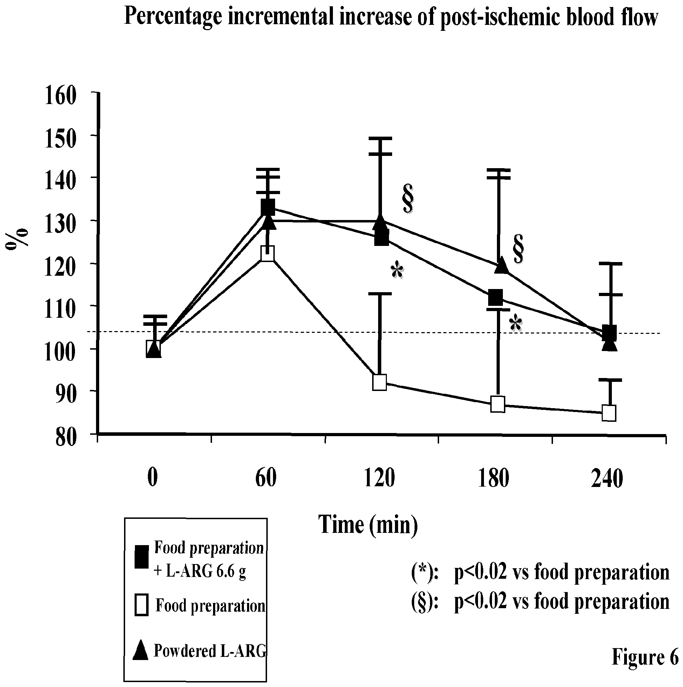

**Figure 6****:** Percentage incremental increase from basal levels in post-ischemic blood flow in subjects receiving the three different treatment conditions. Data are presented as Mean±SD.

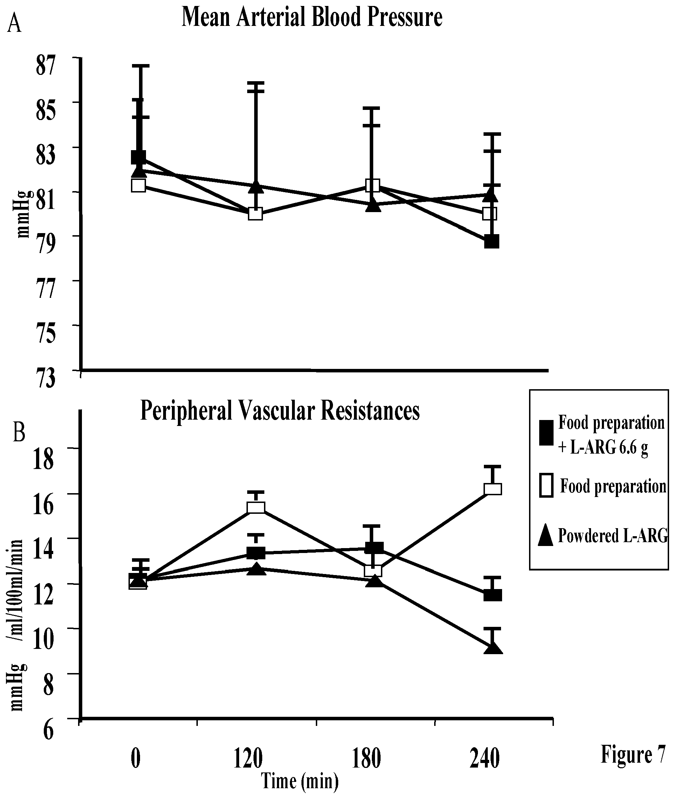

**Figure 7****:** A) Mean arterial blood pressure in subjects receiving the three different treatment conditions and B) Peripheral Vascular Resistances in subjects receiving the three different treatment conditions. Data are presented as Mean±SD.

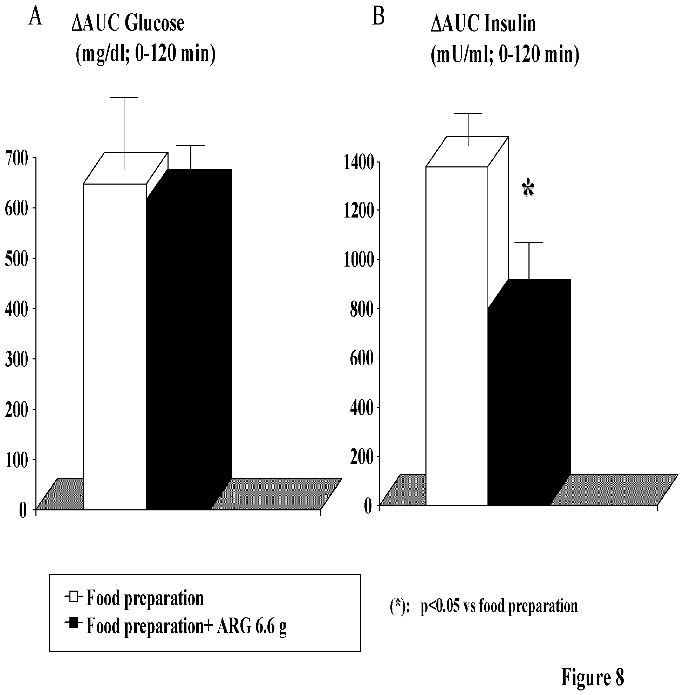

**Figure 8****:** A) Incremental area under the curve for glucose plasma levels (ΔAUC glucose), calculated using the trapezoidal rule, in subjects receiving two different treatment conditions and B) Incremental area under the curve for insulin plasma levels (ΔAUC insulin), calculated using the trapezoidal rule, in subjects receiving two different treatment conditions. Data are presented as Mean±SD.
**Figure 9****:** Study design. Patients were randomized to two groups. One group consumed the food product of the invention, in the form of 6 biscuits containing a total amount of 6.6 g of L-arginine divided into two snacks (one in the morning and one in the afternoon) for 2 weeks followed by the assumption of 6 biscuits having exactly the same composition as the food product of the invention, but without the addition of L-arginine, for 2 weeks, with a 2-week washout in between. The other group consumed these food preparations types in reverse sequence.
   Then the patients were overnight fasted and during OGTT blood samples were collected at baseline and at the end of each food preparation period for serum/plasma biochemistries, and at the same time points body composition and vascular endothelial function and body composition were assessed (Fig. 10-16).
**Figure 10****:** Changes in body weight, fat mass and fat free mass as compared to baseline in subjects receiving the food preparation + 6.6 g of L-arginine (black histograms) and in subjects receiving food preparation without L-arginine (white histogram). To evaluate changes in body weight compositions, patients' response to food preparation interventions was calculated as the difference between the values obtained at the end and at the beginning of the each food preparation intervention period. Data are presented as Mean±SD.
**Figure 11****:** Fasting L-Arginine levels in subjects receiving the food preparation + 6.6 g of L-arginine (black histograms) and in subjects receiving food preparation without L-arginine (white histogram). Data are presented as Mean±SD.
**Figure 12****:** Patterns of plasma NOx levels during OGTT in subjects receiving the food preparation + 6.6 g of L-arginine (black circles) and in subjects receiving food preparation without L-arginine (white circles). Data are presented as Mean±SD.
**Figure 13****:** Comparison of the areas under the curve for NOx (AUC NOx) during the OGTT in subjects receiving the food preparation + 6.6 g of L-arginine (black histograms) and in subjects receiving food preparation without L-arginine (white histogram), calculated using the trapezoidal rule. Data are presented as Mean±SD.
**Figure 14****:** Patterns of plasma cGMP levels during OGTT in subjects receiving the food preparation + 6.6 g of L-arginine (black circles) and in subjects receiving food preparation without L-arginine (white circles). Data are presented as Mean±SD.
**Figure 15****:** Comparison of the incremental areas under the curve for cGMP (ΔAUC cGMP) during OGTT in subjects receiving the food preparation + 6.6 g of L-arginine (black histograms) and in subjects receiving food preparation without L-arginine (white histogram), calculated using the trapezoidal rule. Data are presented as Mean±SD.
**Figure 16****:** Incremental increase from basal levels in post-ischemic blood flow in subjects receiving the two different treatment conditions. Data are presented as Mean±SD.
**Figure 17****:** Patterns of plasma glucose levels during OGTT in subjects receiving the food preparation + 6.6 g of L-arginine (black circles) and in subjects receiving food preparation without L-arginine (white circles). Data are presented as Mean±SD.
**Figure 18****:** Comparison of the areas under the curve for glucose (AUC glucose) during the OGTT in subjects receiving the food preparation + 6.6 g of L-arginine (black histograms) and in subjects receiving food preparation without L-arginine (white histogram), calculated using the trapezoidal rule. Data are presented as Mean±SD.
**Figure 19****:** Patterns of plasma insulin levels during OGTT in subjects receiving the food preparation + 6.6 g of L-arginine (black circles) and in subjects receiving food preparation without L-arginine (white circles). Data are presented as Mean±SD.
**Figure 20****:** Comparison of the areas under the curve for insulin (AUC insulin) during the OGTT in subjects receiving the food preparation + 6.6 g of L-arginine (black histograms) and in subjects receiving food preparation without L-arginine (white histogram), calculated using the trapezoidal rule. Data are presented as Mean±SD.
**Figure 21****:** Matsuda index, an index of insulin sensitivity which take into account insulin and glucose levels during OGTT in subjects receiving the food preparation + 6.6 g of L-arginine (black histograms) and in subjects receiving food preparation without L-arginine (white histogram). Data are presented as Mean±SD.
**Figure 22****:** Proinsulin/insulin ratio as an index of insulin secretion and β-cell function OGTT in subjects receiving the food preparation + 6.6 g of L-arginine (black histograms) and in subjects receiving food preparation without L-arginine (white histogram). Data are presented as Mean±SD.
**Figure 23****:** Fasting triglyceride levels OGTT in subjects receiving the food preparation + 6.6 g of L-arginine (black histograms) and in subjects receiving food preparation without L-arginine (white histogram). Data are presented as Mean±SD.

### Detailed description of the invention

### MATERIALS AND METHODS

### Formulation

The formulation comprises at least 1 g of L-Arginine per single unit. One single unit weight is about 10 g. The invention therefore provides about *10 weight* % *of L-Arginine* (1 g of L-Arginine per unit of 10 g → 10 g of L-Arginine in 100 g of food preparation) in combination with about:
- 20-25 weight % of Mixed cereal flakes (Lameri SpA-S.Bassano-Cr),
- 16-20 weight % of Puffed rice,
- 15-19 weight % of Hazel-nut,
- 11-15 weight % of Orange rind (Cesarin Spa-Verona-Italy)

Mixed cereal flakes are a mixture of corn, oat and whole wheat flakes; they may be manufactured or obtained from any commercial sources.

Puffed rice may be prepared from white or whole rice by high pressure steam treatment, may be manufactured or obtained from any commercial sources.

Finally, hazelnut may be replaced by any dry fruit such as almond, pine nut or other nut and may be used in granulated form, produced or obtained from any commercial sources. In the following table (Table 1) is listed the percentage of ingredients in the formulation of the food product of the invention and its composition (% wet weight) in nutrients (Table 2), assessed by Association of Official Agricultural Chemists (AOAC) methods (2).

**Table 1: Ingredients in the formulation of the food product of the invention**

| **Major ingredients** | **Broad Range % on mix** | **Preferred Range % on mix** |
|---|---|---|
| Arginine Eurosup (L-Arg 58%) | 19-30 | 20-25 |
| Mixed cereal flakes | 20-35 | 24-28 |
| Puffed Rice | 14-25 | 16-20 |
| Hazel-nut | 12-24 | 15-19 |
| Orange rind | 9-18 | 11-15 |
| Water | 2-10 | 4-8 |

Besides the ingredients described above, eccipients of Arginine Eurosup (citric acid, natural flavour on arabica gum, sucralose, acesulfame) are present in order to enhance flavour, usually in an amount that does not exceed 9 weight % .

In the following table (Table 2) is listed the composition (% wet weight) in nutrients of the food product of the invention assessed by Association of Official Agricultural Chemists (AOAC) methods (2).

**Table 2: Composition (% wet weight) in nutrients of the food product of the invention**

| **Biscuits Proximate Composition** | **% ww (preferred composition)** |
|---|---|
| **Water** | **16.9-17.8** |
| **Ash** | **1.33-1.41** |
| **Protein** | **5.0-5.4** |
| **Starch** | **29.0-30.8** |
| **Sugars** | **6.6-6.9** |
| Glucose | 1.8-2.7 |
| Fructose | 0.3-0.5 |
| Lactose | 0.17-0.27 |
| Saccharose | 1.35-1.45 |
| Maltose | 1.8-2.0 |
| **Fat (total)** | **9.6-11.4** |
| **Dietary fiber (total)** | **4.9-6.7** |
| Soluble | 1.2-1.8 |
| Insoluble | 4.4-5.0 |
| **Arginine** | **11.3 -11.5** |

### Preparation of the formulation

The formulation was produced by mixing all ingredients with L-arginine. A minimum of water (about 4-8 % of total weight) is added to support the ingredients' homogenization. Water may be substituted by other liquid such as fruit juice.

For example the formulation of the invention can be made by combining 20% of L-Arginine, Kyowa (Eurosup, Via Novara 4, Castello D'Agogna, PV, Italy) as source of L-Arginine, with 42% of cereal flakes (corn, oat, whole wheat) and 30% of dried un-sugared fruit (cranberries, blueberries, raspberry, blackberry) in order to obtain different flavoring and the desired level of functional substances in the final product.

The blend appears homogeneous, well mixed and lightly damp. Then an aggregation/shaping process is performed by the sonication of the mixture, using in particular a prototype of sonotrode in titanium (20-40 kHz; Branson). A cylindrical mould was used to achieve the shape of the food product; however any mould known in the art is suitable. The process is maintained for 20-2000 milliseconds at a starting temperature of 25-40 °C. Thus there is no temperature change at the inner part of the product till the end of the sonication process. The use of sonication in food processing has been reported in 10 and 22.

To increase the shelf life of the food product, its water content is successively reduced by a batch drying process that can be conducted in a static or in a continuous oven at a temperature less than 60 °C. The food product, ready to eat, is then conveniently packed and stored at room temperature.

The resulting product is storage stable under normal conditions for an extended period of time, has pleasant organoleptic properties, is tasty and provides healthy ingredients (whole cereals) in combination with Arginine.

The number of units taken daily will be about 6, capable to provide the useful functional intake of L-Arginine. One single unit weight is about 10 g and comprises at least 1 g of L-arginine. No undesiderable after taste has been pointed out.

### Clinical study in healthy subjects

In the present invention, the bio-availability and the vascular and metabolic effect of an oral administration of L-arginine (6.6 g) contained in the formulation of the invention (6 biscuits) compared either to the same food preparation (6 biscuits) prepared without the addition of L-arginine or to 6.6 g of powdered L-arginine were evaluated in healthy subj ects.

Seven healthy subjects (2M/5F) participated in the study. Their clinical and metabolic characteristics are reported in Table 3.

**Table 3: Clinical and metabolic characteristics of healthy subjects.**

| | |
|---|---|
| Subjects (n°) | 5F/2M |
| Age (year) | 36±4 |
| Height (cm) | 170±0.04 |
| Weight (kg) | 62±5 |
| BMI (kg/m²) | 21.1±1.7 |
| Systolic BP (mmHg) | 106.7±2 |
| Diastolic BP (mmHg) | 69.5±1.0 |
| Waist (cm) | 74.9±4.4 |
| Hip (cm) | 89.6±4.0 |
| Fasting glucose (mg/dl) | 85.2±3.7 |
| Fasting insulin (µU/ml) | 5.6±1.6 |
| HOMA index | 1.17±0.11 |
| Fasting nitric oxide (µmol/L) | 11.8±2.0 |

A complete medical history and a physical examination including height, weight, waist and hip circumferences and blood pressure measurements were taken for each subject. It was asked to all subjects to follow a 2000 kcal/die standard diet, according to LARN (Livelli di Assunzione Giornalieri Raccomandati di Nutrienti per la Popolazione Italiana) recommendations and to complete a 3 day food diary (two working days and one holiday day) before every test to obtain accurate information on short-term food intake. Food diaries were elaborated with a dedicated software to decode foods (Nutritionist Pro 2.5, Axial System, Stafford, Texas), modified introducing the L-arginine contents, obtained from INRAN and USDA database, in more than 700 different foods items. Analysis of the diaries demonstrated that subjects' diets were balanced since the total caloric intake and the percentages of nutrients consumption in the days before tests were in accordance with LARN recommendations (Figure 1 A). Further, it is possible to estimate an average daily intake of L-arginine of about 2.5 g/day (2.5±0.3 g/day during the three days preceding the first test, 2.6±0.2 g/day during the three days preceding the second test and 2.2±0.5 g/day during the three days preceding the third test, without differences among the three evaluations; Figure 1 B).

Subjects underwent 3 different tests, in random order, with at least a 14-day interval.
▪ The first test consisted in an oral administration of the food product of the invention, in the form of 6 biscuits containing a total amount of 6.6 g of L-arginine. The portion of 6 biscuits further contained a total amount of 21.9 g of carbohydrates (17.9 g of starch and 4.0 g of sugars), 3.6 g of proteins, 7.5 g of fats and 4.3 g of fibers. A fixed amount of 250 ml of natural water was taken with the food preparation. Nutrient composition of the products was assessed by Association of Official Agricultural Chemists (AOAC) methods.
▪ The second test consisted in an oral administration of 6 biscuits having exactly the same composition as the food product of the invention, but without the addition of L-arginine. A fixed amount of 250 ml of natural water was taken with the food preparation.
▪ The third test consisted in the oral administration of 6.6 g of powdered L-arginine diluted in 250 ml of natural water.

The Study Design is represented in Figure 2. After an overnight fast, a 20-gauge plastic cannula (Abbocath T; Abbocath, Ireland LTD, Sling, Ireland) was inserted in a large antecubital vein for intermittent sampling. Samples for the evaluation of glucose, insulin, NOx and L-arginine levels were evaluated at 0, 30, 60, 90, 120, 180 and 240 minutes. c-GMP levels were evaluated at 0, 60, 120, 180 and 240 minutes.

Basal blood pressure was taken in supine position after 10 min of rest, and the mean of two measurements was used as the value, after that blood pressure was also taken at time 0, 120, 180 and 240 minutes. Forearm blood flow (FBF) was measured by strain-gauge venous occlusion plethysmography. Before any measurement was taken, the hand circulation was occluded using a wrist cuff inflated to 240 mmHg. Baseline blood flow was calculated as the mean of at least three values. Reactive hyperaemia (endothelium-dependent vasodilation) was measured after the release of a 5-min arterial occlusion, produced by inflating a standard sphygmomanometer cuff on the upper arm to 100 mmHg above systolic blood pressure (SBP). These measurement were performed at basal and every 60 min until the end of the study. Peripheral resistance was calculated as a ratio between mean blood pressure and the FBF.

### Clinical study in obese subjects with IGT and MS

In the present invention, the chronic (14 days) beneficial effects of an oral administration L-arginine (6.6 g) contained in the formulation of the invention (6 biscuits) were evaluated on body weight composition, amelioration of endothelial function, insulin activity, i.e. on insulin sensitivity and insulin secretion, and lipid levels as compared to the same food preparation (6 biscuits) prepared without the addition of L-arginine for 14 days in obese subjects with IGT and MS.

Fifteen patients with IGT and MS (7M/8F) participated in the study. Their clinical and metabolic characteristics are reported in Table 4.

**Table 4: Clinical and metabolic characteristics of patients with IGT and MS.**

| | |
|---|---|
| **Age (years)** | **62.5 ± 3.5** |
| **Gender M:F** | **8 : 7** |
| **Weight (kg)** | **84.5 ± 4.2** |
| **BMI(kg/m2)** | **30.3 ± 1.5** |
| **Fat Mass (FFM, kg)** | **29.6 ± 3.0** |
| **Free Fat Mass (FM, kg)** | **62.5 ± 3.5** |
| **Waist (cm)** | **M: 108.8 ± 4.0** |
| | **F: 102.0 ± 4.3** |
| **Systolic Blood Pressure (mmHg)** | **121.3 ± 4.0** |
| **Diastolic Blood Pressure (mmHg)** | **76.0 ± 2.0** |
| **Fasting glucose levels (mg/dl)** | **113.2 ± 3.5** |
| **Fasting insulin levels (µU/ml)** | **9.4 ± 2.0** |
| **Total cholesterol levels (mg/dl)** | **160.0 ± 9.1** |
| **HDL cholesterol levels (mg/dl)** | **43.4 ± 3.1** |
| **Trigliceride levels (mg/dl)** | **96.3 ± 13.4** |
| **NOx (µmol/l)** | **17.9 ± 2.8** |
| **cGMP (pmol/mL)** | **7.4 ± 0.9** |
| **Basal forearm blood flow (ml/100 ml/min)** | **2.98± 0.24** |
| **Post-ischemic forearm blood flow (ml/100 ml/min)** | **5.73 ± 0.63** |

This 7-week study enrolled 15 obese subjects with IGT and MS (8 men/7 women, aged 62.5±3.5 years) in a randomized double-blind placebo-controlled crossover design. Two types of food preparations were used: L-arginine (6.6 g) contained in the formulation of the invention (6 biscuits) and the same food preparation (6 biscuits) prepared without the addition of L-arginine, both preparations were packaged identically. The amount of calories derived from the biscuits was included in a 1600 hypocaloric diet.

Patients were randomized to two groups. One group consumed the food product of the invention, in the form of 6 biscuits containing a total amount of 6.6 g of L-arginine divided into two snacks (one in the morning and one in the afternoon) for 2 weeks, followed by the assumption of 6 biscuits having exactly the same composition as the food product of the invention, but without the addition of L-arginine for 2 weeks, with a 2-week washout between the two study periods. During the washout period, a free diet was allowed. The other group consumed these food preparations types in reverse sequence.

A baseline evaluation and oral glucose tolerance test was performed to recruit only patients with IGT and MS. The latter was defined according to ATP III (defined as at least three of the following: waist >102 cm in men and >88 cm in women; triglyceride≥150 mg/dl or patients with specific therapy; HDL cholesterol<40 mg/dl in men and <50 mg/dl in women; systolic blood pressure≥130 mmHg and diastolic blood pressure≥85 mmHg or patients with specific therapy; fasting plasma glucose≥100 mg/dl), namely in the presence of one or more risk factors for type 2 diabetes, including overweight (body-mass index [BMI] >25 kg/m², family history of type 2 diabetes (first degree relatives of patients with type 2 diabetes), and cardiovascular disease. Diagnosis of IGT was based on a fasting plasma glucose tests (FPGT) result of less than 7.0 mmol/L (less than 126 mg/dL) and a plasma glucose value of 7.8 mmol/L (140 mg/dL) or more, but less than 11.1 mmol/L (200 mg/dl) 2 h after the 75 g oral glucose load (OGTT). OGTTs were also repeated at the end of each intervention period. The study design is reported in Figure 9.

Body weight, fat mass and fat free mass distribution was evaluated by bioimpedenziometry using TANITA body fat analyzer (Tanita, Tokyo, Japan).

After overnight fasting and during OGTT, blood samples were collected at baseline and at the end of each food preparation period for serum/plasma biochemistries. In particular, samples for the evaluation of glucose, insulin, NOx and L-arginine levels were evaluated at 0, 30, 60, 90, and 120 minutes. c-GMP levels were evaluated at 0, 60, 90 and 120 minutes. Further, samples for the measurement of plasma glucose and serum insulin levels were drawn at 0, 30, 60, 90, and 120 minutes and fasting proinsulin levels were also evaluated. Insulin sensitivity index (Matsuda index) was calculated according to Matsuda et al. (11) during the OGTT. As an index of insulin secretion and B-cell function, proinsulin/insulin ratio was evaluated (17).

Basal blood pressure was taken in supine position after 10 min of rest, and the mean of two measurements was used as the value. Forearm blood flow (FBF) was measured by strain-gauge venous occlusion plethysmography. Before any measurement was taken, the hand circulation was occluded using a wrist cuff inflated to 240 mmHg. Baseline blood flow was calculated as the mean of at least three values. Reactive hyperaemia (endothelium-dependent vasodilation) was measured after the release of a 5-min arterial occlusion, produced by inflating a standard sphygmomanometer cuff on the upper arm to 100 mmHg above systolic blood pressure (SBP). These measurement were performed at basal and every 60 min until the end of the study.

### Laboratory Measurements

Glucose levels were measured with spectrophotometric methods adapted to Cobas MIRA using commercial kits (ABX, Montpellier, France). Insulin and proinsulin levels were assayed with ELISA kits (Insulin ELISA, Mercodia, Uppsala, Sweden and Proinsulin ELISA, DRG, Marburg, Germany). NOx levels were evaluated through the measurement of metabolic end products, i.e., nitrite and nitrate, using enzymatic catalysis coupled with Griess reaction. c-GMP levels were measured with radioimmunoassay kits (NEN Life Science Products, Boston, MA, USA). L-Arginine were extracted from plasma samples by cation-exchange Strata SCX 100-mg columns (Phenomenex) and assayed by high-performance liquid chromatography.

### Statistical analysis

All values are expressed as Mean±SD at each time interval. To evaluate changes in body weight compositions, patients' response to food preparation interventions was calculated as the difference between the values obtained at the end and at the beginning of the each food preparation intervention period.

Areas and incremental areas under the curve (ΔAUCs) of argininemia, NOx, cGMP, glucose, insulin and proinsulin concentrations during the oral glucose load were calculated by the trapezoidal rule. Data are reported as means±SD. Differences between groups were evaluated by paired Student-T test. All analyses were performed using Statistical Package for Social Science (SPSS) version 15.0 software (SPSS Inc., Chicago, Illinois).

### RESULTS

### Clinical study in healthy subjects

Arginine plasma levels were similar in the group receiving the food product of the invention containing L-Arginine and the group receiving powdered L-Arginine (Figure 3A). In both cases, levels were significantly higher than those of the group receiving the food product not containing L-Arginine, suggesting a complete bio-availability of L-Arginine in the food product of the invention. These results suggest also that the low temperature of the sonication process proposed in this invention prevents the degradation of L-Arginine by Maillard reactions or other heat dependent degradations.

These data were magnified calculating the incremental area under the curve for L-Arginine (ΔAUC L-Arginine). In particular, ΔAUCs L-Arginine were 16736±2611 µmol/l (0-240 min) in the group receiving the food product of the invention, containing 6.6 g of L-Arginine and 15050±1353 µmol/l (0-240 min) in the group receiving powdered L-Arginine. By contrast, it was 258±223 µmol/l (0-240 min) in the group receiving the food preparation not containing L-Arginine (Figure 3B).

To the increased bio-availability of L-Arginine corresponded a significant increase in nitric oxide (NOx) and cGMP levels. In particular, nitric oxide (NOx) and cGMP plasma levels were significantly (p<0.03) higher in the groups receiving the formulation of the invention or powdered L-Arginine as compared to the group receiving the food preparation not containing L-Arginine (Figures 4A and 5A). Similarly, ΔAUC NOx and cGMP were significantly (p<0.04) increased when compared to the group receiving the food product not containing L-Arginine (p<0.04 vs Food preparation, Figure 4B and 5B).

Percentage incremental increase of post-ischemic blood flow significantly (p<0.02) increased in the groups receiving the food product of the invention or powdered L-Arginine as compared to the group receiving the food preparation not containing L-Arginine, suggesting a functional effect of the amino acid even when added to the food product (Figure 6). Further, at 240 minutes mean arterial blood pressure and peripheral vascular resistances slightly declined in the group receiving the food product of the invention without reaching a statistical significance (Figure 7A and B).

At metabolic level, the group receiving the food product of the invention had similar glycemic levels to those receiving the food preparation not containing L-Arginine, but the corresponding insulin plasma levels were significantly (p<0.05) lower in the group receiving the food product of the invention (Figure 8A and B). These data suggest an increased insulin sensitivity associated with the L-Arginine intake, even in healthy subj ects.

### Clinical study in obese subjects with IGT and MS

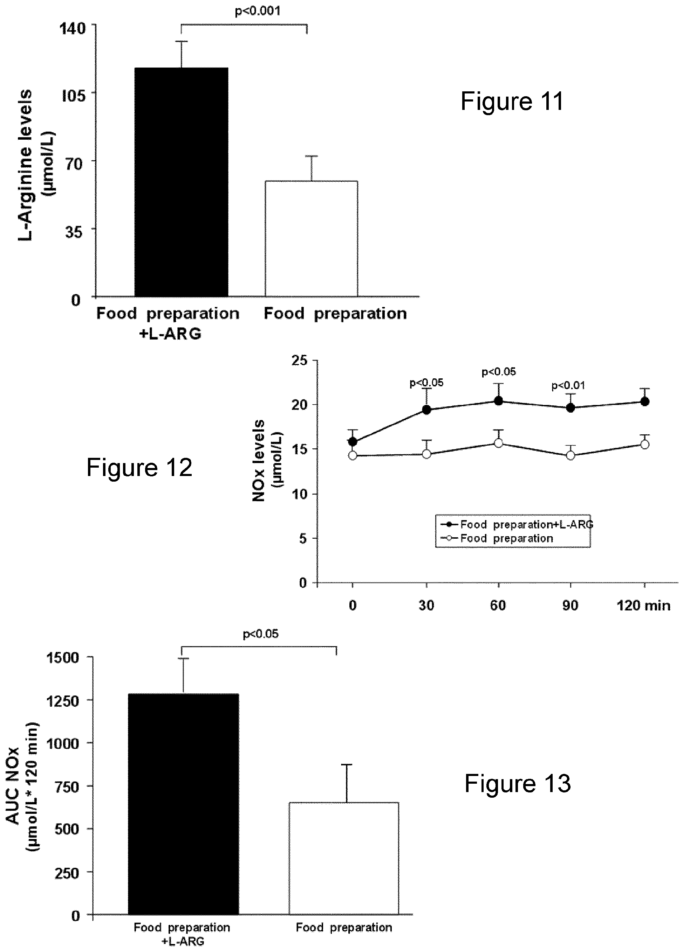

In the group of subjects receiving a 14 days food preparation added with L-arginine a body weight was reduced by 2.57±0.33 kg as compared to a body weight reduction of 1.37±0.34 kg with 14 days food preparation without L-arginine (p<0.05, Figure 10A). Interestingly, during the 14-day food preparation added with L-arginine, nearly all the body weight changes related to a reduction of fat mass (2.02±0.52 kg vs 0.70±0.50 kg with the food preparation without L-arginine; p<0.01, Figure 10B). Conversely, no differences were demonstrated in the loss of fat free between the two groups (figure 10C). Fasting L-Arginine levels were almost doubled in the group receiving the food product of the invention, containing 6.6 g of L-Arginine when compared to the group receiving the food preparation non containing L-Arginine (117.8±26.9 vs 59.3±21.6 µmol/l; p<0.001) (Figure 11).

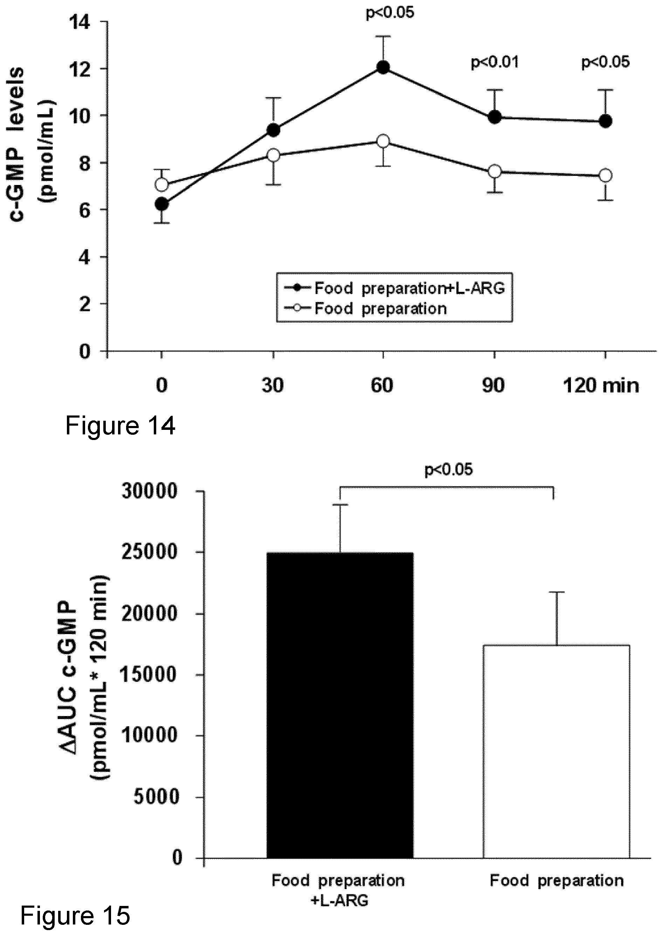

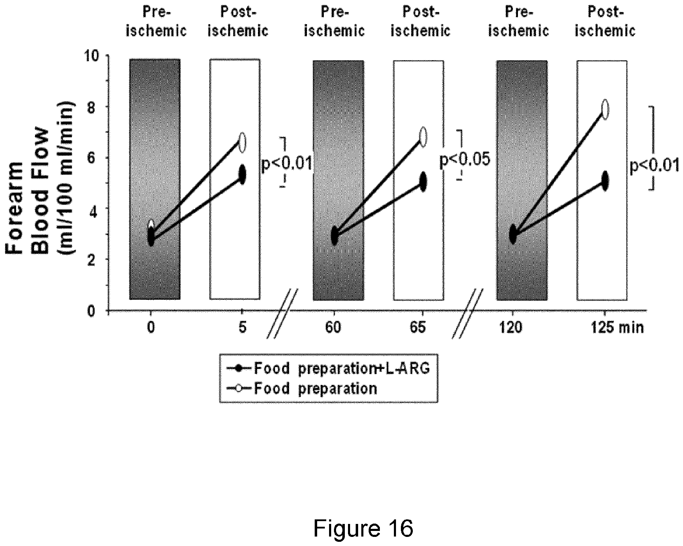

As in the acute study in healthy subjects, the increased bio-availability of L-Arginine corresponded to a significant increase in nitric oxide (NOx) and cGMP levels during OGTT. In particular, nitric oxide (NOx) and cGMP plasma levels were significantly higher in the group receiving the formulation of the invention as compared to the group receiving the food preparation not containing L-Arginine (Figures 12 and 14). Similarly, AUC NOx (1250±200 vs 730±185 µmol/L* 120 min; p<0.05) and ΔAUC cGMP (2495±329 vs 1742±155 pmol/mL* 120 min; p<0.05) were significantly increased when compared to the group receiving the food product not containing L-Arginine (Figure 13 and 15). Post-ischemic blood flow significantly (p<0.01) increased in the group receiving the food product of the invention as compared to the group receiving the food preparation not containing L-Arginine, suggesting a functional effect of the amino acid even when added to the food product (Figure 16).

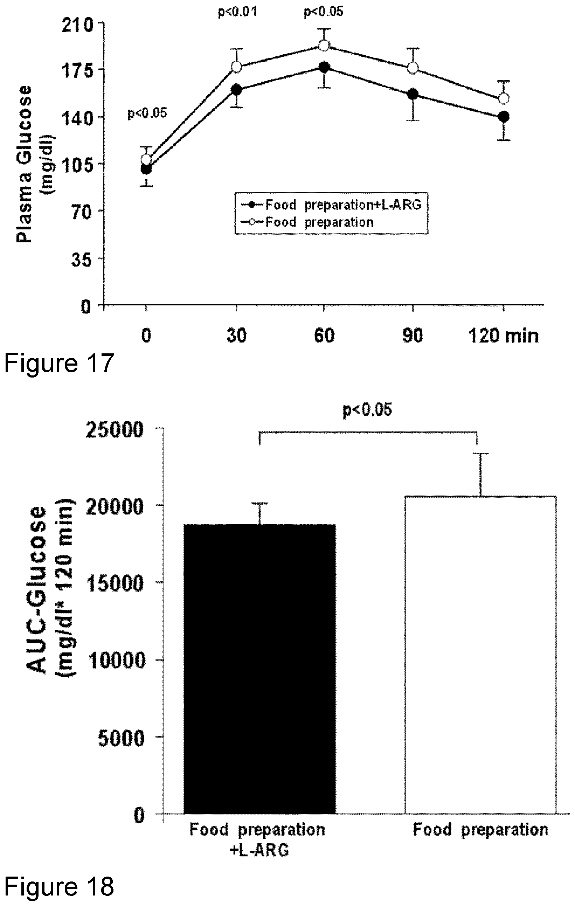

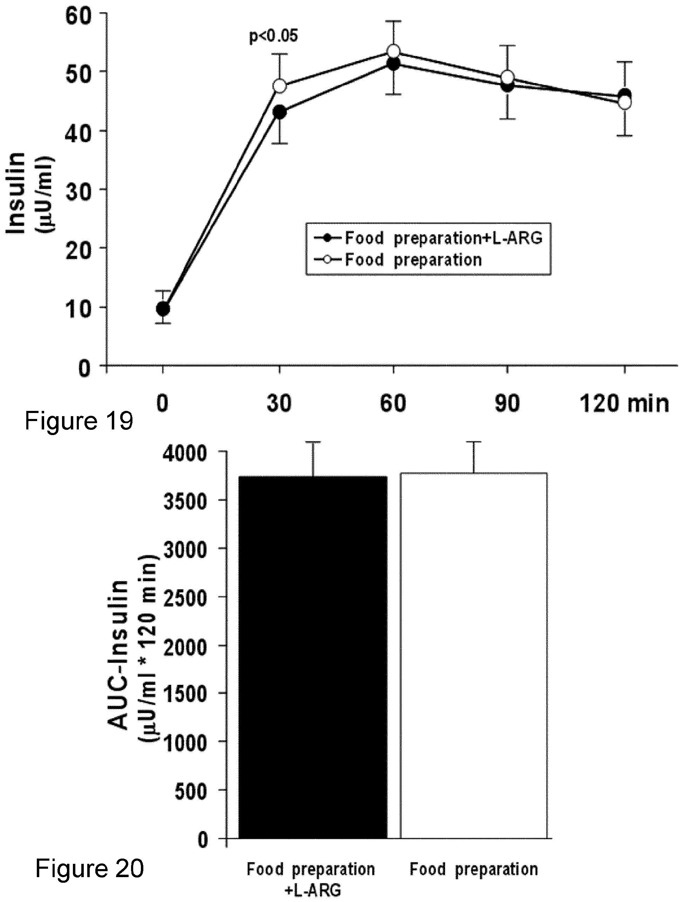

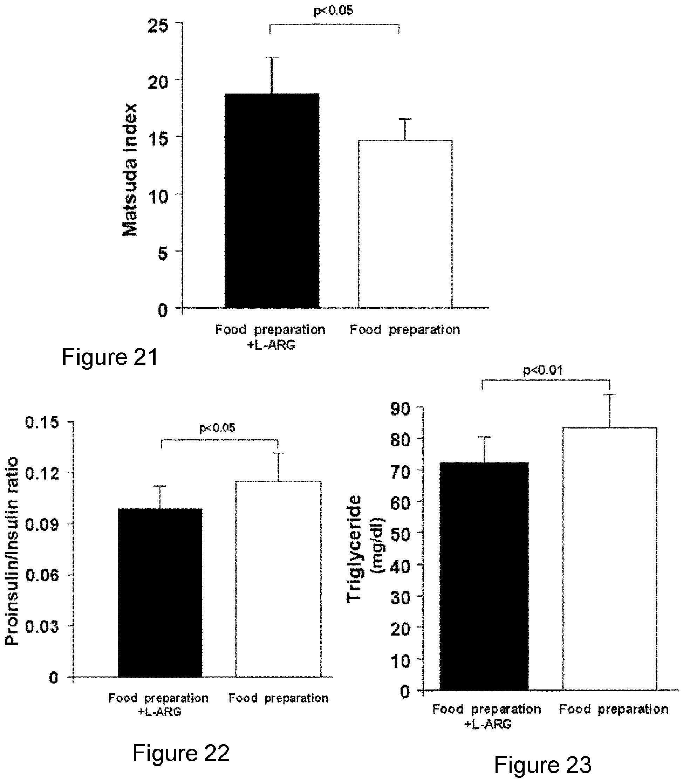

Interestingly, glucose levels (Figure 17) and AUC of glucose (Figure 18) were significantly lower in the group receiving the food product of the invention as compared to the group receiving the food preparation not containing L-Arginine, even if insulin levels were not significantly different (Figure 19 and 20). To prove an increased insulin sensitivity, the authors calculated the Matsuda index, an index of insulin sensitivity, and observed a significantly increased levels in the group receiving the food product of the invention as compared to the group receiving the food preparation not containing L-Arginine (18.7±3.6 vs 14.7±1.6; p<0.05) (Figure 21).

On the contrary, proinsulin/insulin ratio was significantly decreased in the group receiving the food product of the invention as compared to the group receiving the food preparation not containing L-Arginine (Figure 22) and also triglyceride levels were significantly lower in the group receiving the food product of the invention (Figure 23).

### DISCUSSION

In the present invention, an uniformly shaped food product comprising a high content in L-arginine and a low content in sugars and proteins was obtained.

The preparation process of the present invention comprises that all the ingredients (for example puffed rice, whole wheat flakes and granulated hazelnut) are mixed with L-arginine. Then, a step of sonication at low temperature is carried out (22). The present process allows obtaining a uniformly shaped food supplement.

The main key factors of this technological process are:
- no need to raise the temperature up to 82 °C (180 °F) in order to melt the sugars and combine all ingredients as required for the commercial Heartbar®;
- complete *homogeneity, aggregation* and *shape* of the product, achieved by the sonication process.

This leads to a formulation in which amelioration of insulin sensitivity has been detected in normal subjects (see Fig. 8) as well as in obese patients with MS and IGT. The proposed food product of invention contains a reduced amount of calories (by 50%), carbohydrates and sugars (by 87%) as compared to commercially available products (Heartbar®, Cooke Pharma, Belmont, CA, USA) but allows the intake of the same amount of L-Arginine (see list below).

| | ***HeartBars***® ***Size* #*2* (g) 100** | ***Healthy food preparation #6* (g) 60** |
|---|---|---|
| **Calories (kcal)** | **360** | **188** |
| **Protein** | **28 g** | **3.6 g** |
| **L-arginine** | **6.6 g** | **6.6 g** |
| **Total carbohydrates** | **50 g** | **21.9 g** |
| (starch) | | **17.9 g** |
| of which sugars | **30 g** | **4.0 g** |
| **Total fat** | **6 g** | **7.5 g** |
| **Dietary fiber** | **6 g** | **4.3 g** |

Regarding total carbohydrates content, the food product of the invention is advantageous not only for its lower carbohydrate content, but also for the quality of carbohydrate itself. In fact, in line with current dietary guidelines supporting a limited sugars intake up to 12% of daily energy in favour of complex carbohydrates (starch), the optimized invention contains low amount of sugars (about 6.7 weigth %) and mainly starch from whole cereals (about 25-35 weight %). This feature is favourable in the light of the potential lower glycemic impact induced by processed cereal starch.

The beneficial effect of high-starch diet compared to diets high in fructose/sucrose has been demonstrated since the latter accelerates cardiac systolic dysfunction and mortality in hypertension (21). In particular, the beneficial effects of the starch diet may be mediated by activation of cardioprotective pathways (i.e. improved activity of mitochondrial enzymes) and by reduced stimulation of maladaptive cardiac responses activated by fructose diet feeding (6).

Both effects are particularly advantageous since the present food preparation is addressed to subjects with or at risk of cardiovascular disease, endothelial dysfunction, altered blood pressure, Metabolic Syndrome (including patients with insulin resistance syndrome, hyperinsulinemia, a population at highly increased cardiovascular risk), high level of triglycerides, low level of HDL cholesterol, obesity, and impaired glucose tolerance or diabetes.

Metabolic syndrome is a cluster of metabolic abnormalities which includes diabetes or impaired glucose tolerance, hypertension, dyslipidemia, obesity and increased risk of cardiovascular disease (7). It affects one in five people, and prevalence increases with age. Some studies estimate the prevalence in the USA to be up to 25% of the population (3). Metabolic syndrome is also known as metabolic syndrome X, syndrome X, insulin resistance syndrome, Reaven's syndrome (named for Gerald Reaven), and CHAOS (in Australia) (20).

At difference with commercially available health food bars, the food product of the present invention comprises very low levels of fructose (0.6% vs 4-10% of the Heartbar®). In fact, recent evidences in humans also suggest that consuming fructose may have particularly adverse effects on selective deposition of visceral and ectopic fat, lipid metabolism, postprandial hypertriglyceridemia, de novo lipogenesis, blood pressure, and insulin sensitivity, and that this is particularly true in overweight humans (5, 23, 24). Interestingly, doses of 14% of total energy as fructose were able to develop insulin resistance in a period of 9 months in rats (4).

An added value of the present food product is the low content of protein (6.1% vs 20-50%) since it was demonstrated that diets high in protein are associated with an increased diabetes risk, suggesting a potential role of decreased protein content for diabetes prevention (19).

The food product of the present invention is storage stable under normal conditions for an extended period of time, has pleasant organoleptic properties, is tasty and provides healthy ingredients (whole cereals) in combination with Arginine. L-Arginine bio-availability is 100% and this food product shows a beneficial effect on endothelial and vascular function by increasing nitric oxide and its second messenger, cGMP.

An interesting result of the study in obese subjects with IGT and MS was the significant decrease of body weight in the group receiving the product of invention added with L-arginine which was quite completely accounted by a loss of fat mass. These results corroborates previous data in which oral administration of L-arginine added to a structured physical activity and hypocaloric regimen for 21 days was able to decrease body weight mainly as a reduction of fat mass sparing fat free mass in obese type 2 diabetic subjects (9). The strength of the present study is that the loss of body weight was achieved without the help of a structured program of physical activity in patients quite sedentary. Another important result to underline is that obese subjects admitted to take the food preparation with L-arginine lost more weight and fat mass than the same subjects taking the food preparation without the addition of L-arginine.

Similarly, the improvement in insulin sensitivity found in the present study is consistent with previous studies in obese type 2 diabetic patients submitted to L-arg added to a structured physical activity and hypocaloric regimen for 21 days and in cardiopathic subjects with IGT submitted to coronary artery bypass graft (CABG) in which an oral administration of L-arginine for 6 months was able to ameliorate insulin sensitivity without adverse events (9, 8).

Since previous prospective study clearly identified the incapacity of β-cells to compensate for insulin resistance as the key defect leading to type 2 diabetes, from the authors' evaluation of an improvement of proinsulin/insulin ratio (17) they hypothesise that L-arg improves insulin release and ameliorate β-cell machinery for insulin secretion. These data are in line with previous animal and in vitro evidences that pre-treatment with L-arg has a protective action against alloxan-induced β-cell damage (15, 26). Moreover in the same experimental model of alloxan-induced β-cell damage, L-arg induced an increase of insulin immunopositivity in endocrine tissue of diabetic pancreas exposed to alloxan, suggesting the presence of β-cells neogenesis (26).

In the present chronic study in obese subjects with impaired glucose tolerance and metabolic syndrome, the food product added with 6.6 g of L-arginine for 14 days was safe and is useful in decreasing body weight and fat mass, improving endothelial and vascular function, ameliorating glucose metabolism, increasing insulin sensitivity, β-cell function and lipid levels.

### BIBLIOGRAPHIC REFERENCES

1. Abdelhamed AI et al. Am Heart J 145:E15, 2003
2. AOAC. Official methods of analysis. 12th ed. Washington DC, Association of Official Analytical Chemists, 1975.
3. Athyros VG et al. Curr Med Res Opin 21:1157-1159, 2005
4. Blakely SR et al. J Nutr 111:307-314, 1981
5. Brown CM et al. Am J Physiol Regul Integr Comp Physiol 294:R730-R737, 2008
6. Chess DJ et al. Am J Physiol Heart Circ Physiol 293: H1853-H1860, 2007
7. Grundy SM et al. Circulation 112: 2735-2752, 2005
8. Lucotti P et al. Metabolism Clinical and Experimental 58:1270-1276, 2009
9. Lucotti P et al. Am J Physiol Endocrinol Metab 291:E906-E912, 2006
10. Mason TJ et al. Ultrasonics Sonochemistry 3:253-260, 1996
11. Matsuda M et al. Diabetes Care 22:1462-1470, 1999
12. Maxwell AJ et al. WO/1999/059433 Heartbar formulations
13. Maxwell AJ et al. Cardiovascular Drugs and Therapy 14:309-316, 2000
14. Maxwell AJ et al. Vasc Med 5:11-19, 2000
15. Méndez JD et al. Biomed Pharmacol 59:283-289, 2005
16. Monti LD et al. Eur J Clin Invest 38:849-856, 2008
17. Mykkänen L et al. Diabetes 46:1990-1995, 1997
18. Piatti PM et al. Diabetes Care 24:875-880, 2001
19. Piatti PM et al. Circulation. 107:429-436, 2003
20. Reaven GM. Diabetes 37:1595-1607, 1988
21. Sharma N et al. J Hypertens 26:1402-1410, 2008
22. Sluijs I et al. Diabetes Care ;33:43-8, 2010
23. Stanhope KL et al. Curr Opin Lipidol 19:16-24, 2008
24. Stanhope KL et al. J Clin Invest 119:1322-1334, 2009
25. Tiwari BK et al. Food Bioprocess Technol 2:109-114, 2009
26. Vasilijević A et al. J Physiol 584: 921-933, 2007

## Claims

1. An edible formulation comprising the following ingredients, % on mix:
- 19 to 30 weight % of a dietary supplement comprising at least 50 % of L-arginine or a physiologically acceptable salt thereof,
- 20 to 35 weight % of cereal flakes,
- 14 to 25 weight % of puffed brown or white rice,
- 12 to 24 weight % of nuts,
- 9 to 18 weight% of orange rind or dried fruit such as cranberries, blueberries, raspberry, blackberry
- 2 to 10 weight % of water and/or fruit juice.

2. The edible formulation according to claim 1 comprising the following ingredients, % on mix:
- 20 to 25 weight % of a dietary supplement comprising at least 50 % of L-arginine or a physiologically acceptable salt thereof,
- 24 to 28 weight % of cereal flakes,
- 16 to 20 weight % of puffed brown or white rice,
- 15 to 19 weight % of nuts,
- 11 to 15 weight% of orange rind or dried fruit such as cranberries, blueberries, raspberry, blackberry
- 4 to 8 weight % of water and/or fruit juice.

3. The edible formulation according to any one of previous claim being in the form of a food product, preferably in the form of a biscuit or a bar snack.

4. The edible formulation according to any one of previous claim comprising in % wet weight
- at least 10 ww % of L-arginine or a physiologically acceptable salt thereof,
- 5 to 10 ww % of proteins,
- 25 to 32 ww % of starch,
- 6 to 8 ww % of sugars,
- 9 to 12 ww % of total fat,
- 4 to 7 ww % of total dietary fibers.

5. The edible formulation according to claim 4 comprising in % wet weight:
- 11 to 15 ww % of L-arginine or a physiologically acceptable salt thereof,
- 5 to 6 ww % of proteins,
- 29 to 31 ww % of starch,
- 6 to 7 ww % of sugars,
- 9.5 to 11.5 ww % of total fat,
- 4.9 to 6.7 ww % of total dietary fibers.

6. The edible formulation according to claim 4 or 5 comprising in % wet weight:
- 11.3 to 11.5 ww % of L-arginine or a physiologically acceptable salt thereof,
- 5.0 to 5.4 ww % of proteins,
- 29.0 to 30.8 ww % of starch,
- 6.6 to 6.9 ww % of sugars,
- 9.6 to 11.4 ww % of total fat,
- 4.9 to 6.7 ww % of total dietary fibers.

7. The edible formulation according to claim 4 to 6 wherein the starch is from cereals.

8. The edible formulation according to claim 4 to 7 wherein the sugars comprise less than 2 % of fructose.

9. The edible formulation according to any one of previous claim for use as a dietary supplement.

10. The edible formulation according to any one of previous claim for use in the treatment and/or prevention of metabolic syndrome or as a co-adjuvant for the treatment and/or prevention of a metabolic syndrome or for use in the treatment and/or prevention of a pathology wherein the loss of weight and/or fat mass is desirable, wherein said metabolic syndrome includes patients with insulin resistance syndrome, hyperinsulinemia, a population at highly increased cardiovascular risk.

11. The edible formulation according to claim 9 or 10 for use in the treatment and/or prevention of obesity, favoring body weight reduction with positive effects on fat mass within an hypocaloric diet, preferably the obesity is associated with impaired glucose tolerance and metabolic syndrome.

12. The edible formulation according to claim 9 or 10 for use in subjects with or at risk of cardiovascular disease, endothelial dysfunction, altered blood pressure, metabolic Syndrome including patients with insulin resistance syndrome, hyperinsulinemia, a population at highly increased cardiovascular risk, high level of triglycerides, low level of HDL cholesterol, obesity, and impaired glucose tolerance or diabetes.

13. A process for the preparation of the edible formulation according to any one of previous claim comprising:
(a) mixing all the ingredients;
(b) adding water and/or fruit juice;
(c) sonicating the mixture at a temperature between 25 - 40 °C;
(d) optionally, drying the sonicated mixture.

## Patentansprüche

1. Essbare Formulierung, welche die folgenden Bestandteile, als % im Gemisch umfasst:
- 19 bis 30 Gew.-% eines Nahrungsergänzungsmittels, das mindestens 50 % L-Arginin oder ein physiologisch verträgliches Salz davon umfasst,
- 20 bis 35 Gew.-% Getreideflocken,
- 14 bis 25 Gew.-% gepufften braunen oder weißen Reis,
- 12 bis 24 Gew.-% Nüsse,
- 9 bis 18 Gew.-% Orangenschale oder Trockenfrüchte, wie etwa Preiselbeeren, Blaubeeren, Himbeeren, Brombeeren,
- 2 bis 10 Gew.-% Wasser und/oder Fruchtsaft.

2. Essbare Formulierung nach Anspruch 1, welche die folgenden Bestandteile, als % im Gemisch, umfasst:
- 20 bis 25 Gew.-% eines Nahrungsergänzungsmittels, das mindestens 50 % L-Arginin oder ein physiologisch verträgliches Salz davon umfasst,
- 24 bis 28 Gew.-% Getreideflocken,
- 16 bis 20 Gew.-% gepufften braunen oder weißen Reis,
- 15 bis 19 Gew.-% Nüsse,
- 11 bis 15 Gew.-% Orangenschale oder Trockenfrüchte, wie etwa Preiselbeeren, Blaubeeren, Himbeeren, Brombeeren,
- 4 bis 8 Gew.-% Wasser und/oder Fruchtsaft.

3. Essbare Formulierung nach einem der vorherigen Ansprüche, die in Form eines Nahrungsmittelprodukts vorliegt, vorzugsweise in Form eines Kekses oder eines Snackriegels.

4. Essbare Formulierung nach einem der vorherigen Ansprüche, die in % Feuchtgewicht Folgendes umfasst:
- mindestens 10 Gew.-% L-Arginin oder ein physiologisch verträgliches Salz davon,
- 5 bis 10 Gew.-% Proteine,
- 25 bis 32 Gew.-% Stärke,
- 6 bis 8 Gew.-% Zucker,
- 9 bis 12 Gew.-% Gesamtfett,
- 4 bis 7 Gew.-% Gesamtballaststoffe.

5. Essbare Formulierung nach Anspruch 4, die in % Feuchtgewicht Folgendes umfasst:
- 11 bis 15 Gew.-% L-Arginin oder ein physiologisch verträgliches Salz davon,
- 5 bis 6 Gew.-% Proteine,
- 29 bis 31 Gew.-% Stärke,
- 6 bis 7 Gew.-% Zucker,
- 9,5 bis 11,5 Gew.-% Gesamtfett,
- 4,9 bis 6,7 Gew.-% Gesamtballaststoffe.

6. Essbare Formulierung nach Anspruch 4 oder 5, die in % Feuchtgewicht Folgendes umfasst:
- 11,3 bis 11,5 Gew.-% L-Arginin oder ein physiologisch verträgliches Salz davon,
- 5,0 bis 5,4 Gew.-% Proteine,
- 29,0 bis 30,8 Gew.-% Stärke,
- 6,6 bis 6,9 Gew.-% Zucker,
- 9,6 bis 11,4 Gew.-% Gesamtfett,
- 4,9 bis 6,7 Gew.-% Gesamtballaststoffe.

7. Essbare Formulierung nach Anspruch 4 bis 6, wobei die Stärke aus Getreiden stammt.

8. Essbare Formulierung nach Anspruch 4 bis 7, wobei die Zucker weniger als 2% Fructose umfassen.

9. Essbare Formulierung nach einem der vorherigen Ansprüche zur Verwendung als Nahrungsergänzungsmittel.

10. Essbare Formulierung nach einem der vorherigen Ansprüche zur Verwendung bei der Behandlung und/oder Prävention von metabolischem Syndrom oder als Co-Adjuvans zur Behandlung und/oder Prävention eines metabolischen Syndroms oder zur Verwendung bei der Behandlung und/oder Prävention einer Pathologie, bei der ein Verlust von Gewicht und/oder Fettmasse wünschenswert ist, wobei das metabolische Syndrom Patienten mit Insulinresistenzsyndrom, Hyperinsulinämie und eine Population mit stark erhöhtem kardiovaskulärem Risiko einschließt.

11. Essbare Formulierung nach Anspruch 9 oder 10 zur Verwendung bei der Behandlung und/oder Prävention von Fettleibigkeit, die eine Körpergewichtsabnahme mit positiven Auswirkungen auf die Fettmasse innerhalb einer kalorienreduzierten Diät begünstigt, wobei die Fettleibigkeit vorzugsweise mit einer beeinträchtigten Glukosetoleranz und einem metabolischen Syndrom verbunden ist.

12. Essbare Formulierung nach Anspruch 9 oder 10 zur Verwendung bei Patienten mit oder mit einem Risiko für kardiovaskuläre Erkrankung, endotheliale Dysfunktion, veränderten Blutdruck, metabolisches Syndrom, einschließlich Patienten mit Insulinresistenzsyndrom, Hyperinsulinämie, einer Population mit stark erhöhtem kardiovaskulärem Risiko, hohem Spiegel an Triglyceriden, niedrigem Spiegel an HDL-Cholesterin, Fettleibigkeit und gestörter Glukosetoleranz oder Diabetes.

13. Verfahren zur Herstellung der essbaren Formulierung nach einem der vorherigen Ansprüche, das Folgendes umfasst:
(a) Mischen aller Bestandteile;
(b) Zugabe von Wasser und/oder Fruchtsaft;
(c) Ultraschallbehandeln des Gemischs bei einer Temperatur von 25 - 40 °C;
(d) wahlweise Trocknen des ultraschallbehandelten Gemischs.

## Revendications

1. Formulation comestible comprenant les ingrédients suivants, en % du mélange :
- de 19 à 30 % en poids d'un complément alimentaire comprenant au moins 50 % de L-arginine ou d'un sel physiologiquement acceptable de celle-ci,
- de 20 à 35 % en poids de flocons de céréales,
- de 14 à 25 % en poids de riz soufflé blanc ou marron,
- de 12 à 24 % en poids de noix,
- de 9 à 18 % en poids de peau d'orange ou de fruits secs tels que des canneberges, des myrtilles, des framboises, des mûres,
- de 2 à 10 % en poids d'eau et/ou de jus de fruit.

2. Formulation comestible selon la revendication 1, comprenant les ingrédients suivants, en % du mélange :
- de 20 à 25 % en poids d'un complément alimentaire comprenant au moins 50 % de L-arginine ou d'un sel physiologiquement acceptable de celle-ci,
- de 24 à 28 % en poids de flocons de céréales,
- de 16 à 20 % en poids de riz soufflé blanc ou marron,
- de 15 à 19 % en poids de noix,
- de 11 à 15 % en poids de peau d'orange ou de fruits secs tels que des canneberges, des myrtilles, des framboises, des mûres,
- de 4 à 8 % en poids d'eau et/ou de jus de fruit.

3. Formulation comestible selon l'une quelconque des revendications précédentes, é tant sous la forme d'un produit alimentaire, de préférence sous la forme d'un biscuit ou d'une barre de type en-cas.

4. Formulation comestible selon l'une quelconque des revendications précédentes, comprenant en % en poids humide :
- au moins 10 % en poids humide de L-arginine ou d'un sel physiologiquement acceptable de celle-ci,
- de 5 à 10 % en poids humide de protéines,
- de 25 à 32 % en poids humide d'amidon,
- de 6 à 8 % en poids humide de sucres,
- de 9 à 12 % en poids humide de matières grasses totales,
- de 4 à 7 % en poids humide de fibres alimentaires totales.

5. Composition comestible selon la revendication 4, comprenant en % en poids humide :
- de 11 à 15 % en poids humide de L-arginine ou d'un sel physiologiquement acceptable de celle-ci,
- de 5 à 6 % en poids humide de protéines,
- de 29 à 31 % en poids humide d'amidon,
- de 6 à 7 % en poids humide de sucres,
- de 9,5 à 11,5 % en poids humide de matières grasses totales,
- de 4,9 à 6,7 % en poids humide de fibres alimentaires totales.

6. Composition comestible selon la revendication 4 ou 5, comprenant en % en poids humide :
- de 11,3 à 11,5 % en poids humide de L-arginine ou d'un sel physiologiquement acceptable de celle-ci,
- de 5,0 à 5,4 % en poids humide de protéines,
- de 29,0 à 30,8 % en poids humide d'amidon,
- de 6,6 à 6,9 % en poids humide de sucres,
- de 9,6 à 11,4 % en poids humide de matières grasses totales,
- de 4,9 à 6,7 % en poids humide de fibres alimentaires totales.

7. Composition comestible selon l'une quelconque des revendications 4 à 6, dans laquelle l'amidon provient de céréales.

8. Composition comestible selon l'une quelconque des revendications 4 à 7, dans laquelle les sucres comprennent moins de 2 % de fructose.

9. Composition comestible selon l'une quelconque des revendications précédentes, destinée à être utilisée en tant que complément alimentaire.

10. Composition comestible selon l'une quelconque des revendications, destinée à être utilisée dans le traitement et/ou la prévention d'un syndrome métabolique ou en tant que co-adjuvant pour le traitement et/ou la prévention d'un syndrome métabolique ou destinée à être utilisée dans le traitement et/ou la prévention d'une pathologie dans laquelle la perte de poids et/ou de masse graisseuse est souhaitable, ledit syndrome métabolique comprenant des patients souffrant d'un syndrome d'insulinorésistance, d'une hyperinsulinémie, une population présentant un risque cardiovasculaire très élevé.

11. Formulation comestible selon la revendication 9 ou 10, destinée à être utilisée dans le traitement et/ou la prévention de l'obésité, favorisant une réduction du poids corporel avec des effets positifs sur la masse graisseuse dans un régime hypocalorique, de préférence l'obésité est associée à une tolérance au glucose altérée et un syndrome métabolique.

12. Formulation comestible selon la revendication 9 ou 10, destinée à être utilisée chez des sujets souffrant ou à risque de développer une maladie cardiovasculaire, un dysfonctionnement endothélial, une pression artérielle modifiée, un syndrome métabolique, y compris les patients souffrant d'un syndrome d'insulinorésistance, d'une hyperinsulinémie, une population présentant un risque cardiovasculaire très élevé, un taux élevé de triglycérides, un faible taux de cholestérol HDL, une obésité et une tolérance au glucose altérée ou un diabète.

13. Procédé de préparation de la formulation comestible selon l'une quelconque des revendications précédentes comprenant :
(a) le mélange de tous les ingrédients ;
(b) l'ajout d'eau et/ou de jus de fruit ;
(c) la sonication du mélange à une température comprise entre 25 et 40 °C ;
(d) facultativement, le séchage du mélange soniqué.
